# EUROPÄISCHE PATENTSCHRIFT

(11) **EP 0 522 269 B1**
(45) Veröffentlichungstag und Bekanntmachung des Hinweises auf die Patenterteilung: **12.01.2000**
(21) Anmeldenummer: 92108559.3
(22) Anmeldetag: 21.05.1992
(51) Int. Cl.: C12N 9/98

(54) **Verfahren zur Herstellung von Enzymzubereitungen**
Method for producing enzyme composition
Méthode pour la préparation de compositions enzymatiques

(30) Priorität: 12.06.1991 DE 4119281
(43) Veröffentlichungstag der Anmeldung: 13.01.1993
(73) Patentinhaber: BASF Aktiengesellschaft, 67063 Ludwigshafen (DE)
(72) Erfinder: Bewert, Wolfgang, Dr., W-6710 Frankenthal (DE); Schwarz, Gerhard, Dr., W-6721 Harthausen (DE)

(56) Entgegenhaltungen:
- EP-A- 0 074 050
- EP-A- 0 263 372
- EP-A- 0 294 520
- GB-A- 1 397 410
- US-A- 4 233 405
- US-A- 4 617 272
- PATENT ABSTRACTS OF JAPAN vol. 9, no. 166 (C-290)(1889) 11. Juli 1985

## Beschreibung

Die Erfindung betrifft ein verbessertes Verfahren zur Herstellung von trockenen, freifließenden, stabilen Enzymzubereitungen durch Versprühen einer wäßrigen Enzymdispersion in einer Sprüheinrichtung.

Die Isolierung von Enzymen geschieht üblicherweise als wäßrige Lösung. In dieser Form verlieren die Enzyme sehr schnell ihre Aktivität und lassen sich nur unter großem Aufwand lagern und transportieren. Deshalb ist es wünschenswert, trockene Zubereitungen von Enzymen herzustellen, die das Enzym unter möglichst geringem Aktivitätsverlust in konzentrierter Form enthalten. Außerdem sollen diese Zubereitungen aus Teilchen mit gut ausgebildeter Oberfläche in einer Korngröße von 50 bis 600 µm bestehen, so daß in der weiterverarbeitenden pharmazeutischen Industrie, Nahrungsmittel- und Futtermittel industrie eine homogene Mischung dieser Produkte mit anderen Stoffen oder mit Nahrungs- und Futtermitteln möglich ist.

Es gibt verschiedene Sprühverfahren, um Wasser aus Enzym-haltigen wäßrigen Medien zu entfernen.

In der amerikanischen Patentschrift 4,617,272 werden Enzym-haltige Medien auf in einem Fließbett erhitzte Inertpartikel aufgesprüht.

Als geeignete Inertpartikel werden Polyolefine, Polycarbonate, Polymethylmethacrylate oder Polystyrol genannt.

Ein Nachteil dieses Verfahrens ist, daß die mittels dieser Inertpartikel hergestellten Enzym-Trockenpulver nicht in der Nahrungs- und Futtermittelindustrie eingesetzt werden können.

Nach einem anderen Verfahren, das in dem Wirtschaftspatent DD 263 790 beschrieben ist, werden Milchgerinnungsproteasenprodukte hergestellt, indem man wäßrige Proteaselösungen auf Trägerstoffe, die sich in einem Wirbelschichtgranulator befinden, aufsprüht. Als Trägerstoffe werden Magermilchpulver und/oder dextrinhaltige Stoffe beschrieben.

Obwohl die erhaltenen Produkte gute Enzym-Stabilität und Rieselfähigkeit besitzen und die verwendeten Trägerstoffe physiologisch verträglich sind, hat dieses Verfahren den Nachteil, daß die bis zu 10fache Menge an Trägerstoffen, bezogen auf Enzym-Feststoff, eingesetzt werden muß.

EP 074050 beschreibt ein Verfahren zur Herstellung von Trockenpulver aus öllöslichen Stoffen durch Versprühen einer Kolloiddispersion unter Zugabe von Sprühhilfsmitteln.

US 4, 233, 405 beschreibt ein Verfahren zur Sprühtrocknung von Enzymen, bei dem die Enzymlösung mit wasserunlöslichen Salzen gemischt und in einem heißen Luftstrom getrocknet werden.

Der Erfindung lag daher die Aufgabe zugrunde, ein Verfahren vorzuschlagen, das es gestattet, wäßrige Enzymdispersionen in trockene, stabile Enzymzubereitungen zu überführen, die in der Nahrungs- und Futtermittelindustrie eingesetzt werden können.

Es wurde nun gefunden, daß das eingangs definierte Verfahren zu besonders gut geeigneten Enzymzubereitungen führt, wenn die gegebenenfalls Zusatzstoffe enthaltende wäßrige Enzymdispersion unter Mitverwendung von 5 bis 60 Gew.-%, bezogen auf den Enzym-Feststoffgehalt der Dispersion, eines Sprühhilfsmittels, bestehend aus hydrophober Kieselsäure bei 0 bis 50°C versprüht und die so erhaltenen, mit Sprühhilfsmittel beladenen Teilchen getrocknet werden.

Als Enzyme, die in diesem Verfahren verwendet werden können, kommen Oxidoreduktasen, Transferasen, Hydrolasen, Lyasen, Isomerasen und Ligasen in Betracht.

In der Nahrungs- und Futtermittel industrie finden speziell Hydrolasen Verwendung, die die komplexen Nahrungsbestandteile in physiologisch verwertbare Einheiten spalten.

Bei den Hydrolasen werden die Peptidbindungen-spaltenden Enzyme (Proteasen und Peptidasen) wie z.B. Chymotrypsin, Trypsin, Papain, Pepsin, Collagenase, Carboxypeptidase oder Ester-spaltende Enzyme (Lipasen, Phosphatasen, Sulfatasen) wie z.B. Pankreatin, alkalische oder saure Phosphatase, sowie Phytase bevorzugt verwendet.

Die Herkunft der Enzyme spielt dabei keine Rolle. So können die Enzyme beispielsweise aus Tieren oder Pflanzen oder Tier- oder Pflanzenteilen isoliert werden. Ebenso können Enzyme, die aus Mikroorganismen wie z.B. Bakterien oder Hefen isoliert worden sind, verwendet werden. Auch Enzyme, die mittels gentechnisch veränderter Organismen gewonnen wurden kommen in Betracht.

Als Zusatzstoffe, die der wäßrigen Enzymdispersion beigegeben werden können, kommen bevorzugt physiologisch unbedenkliche Materialien, wie sie üblicherweise in der Futtermittelindustrie Verwendung finden, in Betracht.

Hierzu zählen Polysaccharide wie Zelluloseverbindungen, Pektine und Stärken unterschiedlicher Herkunft, filmbildende Kolloide wie Gelatine, Casein oder Albumin, Mono- oder Disaccharide wie Glucose, Fructose, Lactose oder Saccharose, oder pflanzliche Produkte wie beispielsweise Weizengrießkleie oder Sojamehl.

Weiterhin können als Zusatzstoffe anorganische Materialien wie Calciumcarbonat, Tonerden, verschiedene Formen von gefällten oder mineralischen Kieselsäuren und Silikaten aber auch Produkte tierischer Herkunft, wie z.B. Eierschalenmehl, verwendet werden. Außerdem können noch weitere Zusätze wie Emulgatoren, Antioxidantien oder Konservierungsmittel verwendet werden.

Die Menge der angewandten Zusatzstoffe beträgt in der Regel 5 bis 90 Gew.-%, bevorzugt 20 bis 80 Gew.-%, bezogen auf Enzym-Feststoff.

Das erfindungsgemäße Verfahren kann folgendermaßen ausgeführt werden:

Das Sprühhilfsmittel wird zusammen mit Luft oder einem Inertgas durch Versprühen in eine Sprüheinrichtung, vorzugsweise einen Sprühturm, eingebracht. Die Zufuhr des Sprühhilfsmittels erfolgt zweckmäßig oberhalb des Zerstäubungsaggregats.

Die wäßrige Enzymdispersion kann unter Druck durch Düsen in den mit Sprühhilfsmittel beladenen Sprühraum eingebracht werden. Es ist aber auch möglich, die wäßrige Enzymdispersion auf schnell rotierende Zerstäuberscheiben fließen zu lassen. Die Ausbildung des Zerstäubungsaggregats hat keinen entscheidenden Einfluß auf das Produkt.

Der sich ausbildende Sprühkegel enthält eine Vielzahl kleiner Tröpfchen, die anschließend durch Wasserentzug in eine trockene Enzymzubereitung überführt werden. Die Trocknung geschieht zweckmäßigerweise unmittelbar nach der Versprühung. Geeignet ist hierfür die Verdunstungstrocknung, bei der das wasser aus den Tröpfchen mit Hilfe eines angewärmten Luft- oder Inertgasstroms entfernt wird.

Besonders bevorzugt kann zur Trocknung ein Wirbelbett verwendet werden, das sich unterhalb des Zerstäubungsaggregats befindet.

Bei besonders oxidationsempfindlichen Enzymen wird die Verwendung von Inertgas, wie z.B. Stickstoff, bei der Versprühung und der Trocknung bevorzugt.

Die mit diesem Verfahren hergestellten Enzymzubereitungen zeichnen sich durch ihre gute Stabilität und durch einen geringen Restfeuchtegehalt von weniger als 10 %, bezogen auf Festsubstanz, aus.

Die Temperatur der zu zerstäubenden Dispersion soll 0 bis 50°C betragen. Bei Enzymen, die leicht durch Wärme inaktiviert werden, werden bevorzugt Temperaturen von 0 - 20°C verwendet, bei Hitze-stabilen Enzymen wird eine Temperatur von 20 bis 50°C bevorzugt verwendet. Ob ein Enzym Hitze-labil oder Hitze-stabil ist, kann der Fachmann aus Monographien wie z.B. "Enzyme Handbook", Schomburg, Salzmann (Ed.), Springer Verlag 1990, entnehmen.

Als Sprühhilfsmittel kommen silanisierte Kieselsäuren, wie sie in "Die Mühle und Mischfuttertechnik" 114, 3 (1977) beschrieben sind in Betracht.

Die Gewichtsmenge an Sprühhilfsmittel beträgt 5 bis 60 Gew.-%, bevorzugt 20 bis 50 Gew.-%, bezogen auf Enzym-Feststoff.

Der große Vorteil des neuen Verfahrens besteht darin, daß nicht mehr durch große Mengen an Hilfspulver soviel Wasser entzogen werden muß, daß eine Erstarrung der Tröpfchen erfolgt. Der während des Sprühens erzeugte dünne hydrophobe Film des Sprühhilfsmittels stabilisiert die Partikel so weit, daß ein Zusammenlaufen der Partikel bei Berührung im nicht-erstarrten Zustand verhindert wird, so daß die direkte Trocknung z.B. auf einem sich anschließenden Wirbelbett-Trockner möglich ist.

Durch das direkte Einbringen des Sprühhilfsmittels in die Sprühzone wird die mechanische Beanspruchung der Partikel weitgehend vermieden, die beispielsweise von einem Träger-gefüllten Fließbett ausgeht.

Im einzelnen ist das neue Verfahren in den folgenden Beispielen beschrieben.

### Beispiel 1

300 g wäßriges Phytase-Ultrafiltrat mit einem Feststoffgehalt von 45 g (Enzymgehalt ca. 50 %) und einer Aktivität von 2200 FTU/ml wurden mit 165,8 g Sojamehl und 50 ml Wasser vermischt. Die erhaltene Suspension wurde mit einer Temperatur von 45°C und einem Sprühdruck von 6 bar mit Hilfe einer Einstoffdüse mit einem Durchmesser von 0,8 mm in Luft versprüht, die mit fein verteilter hydrophober Kieselsäure beladen war. Das dabei erhaltene feuchte Produkt wurde in einem Wirbelbett bei einer Umgebungstemperatur von 22°C unter Stickstoffatmosphäre auf einen Restwassergehalt von 4,8 % getrocknet. Bei diesem Trocknungsprozeß wurde gleichzeitig die nicht an das Enzymtrockenpulver gebundene, hydrophobe Kieselsäure ausgetragen und mit Hilfe eines Zyklons abgeschieden. Man erhielt 213 g eines gut rieselfähigen Pulvers mit einem SiO₂-Gehalt von 4,8 % und einem Wirkstoffgehalt von 3,5 · 10⁶ FTU/kg.

### Beispiel 2

300 g wäßriges Phytase-Ultrafiltrat wie in Beispiel 1 wurde mit 155 g gefälltem Calciumcarbonat gemischt und bei einer Temperatur von 45°C und einem Sprühdruck von 6 bar in Luft, die mit hydrophober Kieselsäure beladen war, versprüht. Nach dem Trocknen im Wirbelbett unter Stickstoffatmosphäre bei einer Temperatur von 25°C bis zu einem Restwassergehalt von 1,5 % erhielt man 210 g eines gut rieselfähigen Pulvers mit einem SiO₂-Anteil von 4,7 % und einem Wirkstoffgehalt von 3,5 · 10⁶ FTU/kg.

### Beispiel 3

500 g eines wäßrigen Phytase-Ultrafiltrats mit einem Feststoffgehalt von 75 g und einer Aktivität von 2600 FTU/ml wurden ohne Zusatzstoffe bei einer Temperatur von 45°C wie in Beispiel 1 beschrieben versprüht und anschließend getrocknet. Man erhielt 240 g eines gut rieselfähigen Pulvers mit einem Restwassergehalt von 7,4 % und einem SiO₂-Anteil von 7,9 %. Der Wirkstoffgehalt betrug 13,1 · 10⁶ FTU/kg.

## Patentansprüche

1. Verfahren zur Herstellung von trockenen, freifließenden, stabilen Enzymzubereitungen durch Versprühen von gegebenenfalls Zusatzstoffe enthaltenden wäßrigen Enzymdispersionen in einer Sprüheinrichtung, dadurch gekennzeichnet, daß man die Enzymdispersion unter Mitverwendung von 5 bis 60 Gew.-%, bezogen auf den Enzym-Feststoffgehalt der Dispersion, eines Sprühhilfsmittels, bestehend aus hydrophober Kieselsäure bei 0 bis 50°C versprüht und die so erhaltenen, mit Sprühhilfsmittel beladenen Teilchen trocknet.

2. Verfahren nach Anspruch 1, dadurch gekennzeichnet, daß als Enzym eine Hydrolase verwendet wird.

3. Verfahren nach Anspruch 1 und 2, dadurch gekennzeichnet, daß als Enzym Phytase verwendet wird.

## Claims

1. A process for the production of dry, free-flowing, stable enzyme preparations by spraying aqueous enzyme dispersions, which may contain additives, in a spraying apparatus, which comprises the enzyme dispersion being sprayed in the presence of from 5 to 60% by weight, based on the content of enzyme in the dispersion, of a spray auxiliary composed of hydrophobic silica at from 0 to 50°C, and drying the resulting particles loaded with spray auxiliary.

2. A process as claimed in claim 1, wherein a hydrolase is used as enzyme.

3. A process as claimed in claim 1 or 2, wherein phytase is used as enzyme.

## Revendications

1. Procédé pour la préparation de compositions enzymatiques sèches, stables, qui s'écoulent bien, par pulvérisation de dispersions aqueuses d'enzymes contenant le cas échéant des additifs dans un dispositif de pulvérisation, caractérisé par le fait que l'on pulvérise la dispersion d'enzymes avec utilisation conjointe de 5 à 60% en poids, par rapport aux enzymes sèches de la dispersion, d'un produit auxiliaire de dispersion consistant en une silice hydrophobe, à des températures de 0 à 50°C, puis on sèche les particules ainsi obtenues, chargées du produit auxiliaire de pulvérisation.

2. Procédé selon la revendication 1, caractérisé par le fait que l'enzyme utilisée est une hydrolase.

3. Procédé selon les revendications 1 et 2, caractérisé par le fait que l'enzyme utilisée est une phytase.
